**Europäisches Patentamt**

**European Patent Office**

**Office européen des brevets**

(19)

(11) Veröffentlichungsnummer: **0 029 893**

**B1**

(12) **EUROPÄISCHE PATENTSCHRIFT**

(45) Veröffentlichungstag der Patentschrift:
20.05.87

(21) Anmeldenummer: **80106066.6**

(22) Anmeldetag: **07.10.80**

(51) Int. Cl.⁴: **C 07 G 17/00,** C 07 K 3/02,
C 07 K 3/20, C 07 K 3/28,
C 07 K 15/06, C 12 P 19/34,
C 12 P 21/00, A 61 K 35/00

(54) **Verfahren zur Anreicherung tumorhemmender Wirkfaktoren.**

(30) Priorität: **02.11.79 DE 2944277**
**02.11.79 DE 2944278**

(43) Veröffentlichungstag der Anmeldung:
**10.06.81 Patentblatt 81/23**

(45) Bekanntmachung des Hinweises auf die Patenterteilung:
**20.05.87 Patentblatt 87/21**

(84) Benannte Vertragsstaaten:
**AT BE CH DE FR GB IT LI LU NL SE**

(56) Entgegenhaltungen:
DE-A-1 090 821
DE-A-2 616 984

NATURE, Band 271, 16. Februar 1978, Seiten 622-624, London, GB, J. DE MAEYER-GUIGNARD et al.: "Purification of mouse interferon by sequential affiniy chromatography on poly(U)- and antibody-agarose columns"
PHARMACIA FINE CHEMICALS, "Affinity Chromatography", Juni 1979, Ljungföretagen AB, Orebro, SE
CHEMICAL ABSTRACTS, Band 81, Nr. 15, 14. Oktober 1974, Seite 134, Nr. 87312x, Columbus, Ohio, U.S.A., H. SYSKA et al.: "New method of preparation of troponin I (inhibitory protein) using affinity chromatography. Evidence for three different forms of troponin I in Striated muscle"

(73) Patentinhaber: **Theurer, Karl, Prof. Dr., Brunnwiesenstrasse 21, D-7302 Ostfildern 1 (DE)**

(72) Erfinder: **Theurer, Karl, Prof. Dr., Brunnwiesenstrasse 21, D-7302 Ostfildern 1 (DE)**

(56) Entgegenhaltungen: (Fortsetzung)
CHEMICAL ABSTRACTS, Band 90, Nr. 3, 15. Januar 1979, Seite 451, Nr. 20618u, Columbus, Ohio, U.S.A., N. ORSI et al.: "Affinity chromatography on concanavalin A-sepharose column of human serum lipo-proteins inhibiting rubella virus hemagglutination"
BIOLOGICAL ABSTRACTS, Band 65, Nr. 12, 1978, Nr. 70794, V.B. HATCHER et al.: "A cytotoxic proteinase isolated from human lymphocytes"

## Beschreibung

Die Erfindung betrifft ein Verfanren zur Gewinnung von tumorhemmenden Wirkfaktoren aus einem Substrat aus Zellhomogenisaten von Organgeweben und/oder von Körperflüssigkeiten, insbesondere die Gewinnung von sterilen Wirkfaktoren, die zur Innibition von Stoffwechselvorgängen in Tumorzellen verwendbar sind.

Aufgrund eigener Versuchsergebnisse über die Beeinflussung des Wachstums und des Stoffwechsels von menschlichen Gewebezellen in Zellkulturen durch Behandlung mit makromolekularen Organextrakten (V. Paffenholz und K. Theurer: Einfluß von makromolekularen Organsubstanzen auf menschlicne Zellen in vitro: I. Diploide Kulturen, II. Tumorzellkulturen: Der Kassenarzt H. 27, S. 5218-26, 1978; H. 19, S. 1876-1887, 1979) wurde festgestellt, daß eine Hemmwirkung auf Tumorzellen und heteroploide Zellen durch höhere Verdünnungen in einer Konzentration von $10^{-6}$ bis $10^{-9}$ g/ml Kulturmedium und eine Stimulierung von gesunden Normalzellen durch höhere Konzentrationen zwiscnen $10^{-3}$ bis $10^{-6}$ g/ml Kulturmedium zu erzielen war. Die Wirkfaktoren wurden als Proteine bzw. Peptide identifiziert.

Aufgrund dieser Unterschiede war zu vermuten, daß es sich um unterschiedliche Faktoren zur Hemmung und Stimulierung mit unterschiedlichen Angriffspunkten handeln würde (K. Theurer: Prophylaxe und makromolekularen Organextrakten: Krebsgeschehen 4, 80-86, 1980). Dies führte zur Entwicklung des vorliegenden Verfahrens zur Trennung der hemmenden und stimulierenden Faktoren aus Zellhomogenisaten. Ergebnisse von S.R. Burzynsky, K. Stolzmann, B. Szopa, E. Stolzmann und O.P. Kaltenberg: Antineoplaston A in Cancer Therapy: Physiological Chemistry and Physics 9, 485-500 (1977)) zeigen, daß solche tumorhemmenden Faktoren im menschlichen Harn von gesunden Individuen enthalten sind und aus diesen gewonnen werden können. Ebenso ist auch bereits bekannt, daß in dem Blut von gesunden Individuen tumorhemmende Faktoren enthalten sind.

Ziel der Erfindung ist es, die tumorhemmenden Wirkfaktoren aus den oben genannten Organextrakten oder Körperflüssigkeiten selektiv anzureichern und von unerwünschten Begleitsubstanzen zu befreien, um sie der wissenschaftlichen, therapeutischen und industriellen Verwertung zugänglich zu machen.

Gemäß der Erfindung wird das in wässeriger Form vorliegende Substrat, das von partikulären Begleitstoffen frei ist bzw. befreit ist, mit Hilfe eines biologischen Trennmittels, bei dem mindestens eine Nukleinsäure an eine Trägersubstanz gekoppelt ist, selektiv aufgetrennt, wobei zunächst nicht affine Komponenten und anschließend die die affinen Wirkfaktoren enthaltenden Fraktionen eluiert werden. Zur Gewinnung des die affinen Wirkstoffe enthaltenden Eluats werden besondere Hilfsmittel eingesetzt, die die Wirkstoffe vom Trennmittel ablösen. Das so erhaltene Eluat kann einer weiteren Fraktionierung unterworfen werden, wobei physikalisch-chemische Fraktionierungen bevorzugt sind.

Die Auftrennung des Substrates erfolgt somit erfindungsgemäß durch Affinitätschromatographie bzw. durch eine Kombination von Affinitätschromatographie und Elektrophorese. Dabei kann die Elektrophorese nach Art der "Free-flow-Electrophoresis" durchgeführt werden. Die Affinitätschromatographie erlaubt eine selektive Abtrennung der gewünscnten Wirkfaktoren, wenn die Nukleinsäuren, die mit der Trägersubstanz unter Bildung des Trennmittels gekoppelt werden, in Abhängigkeit von den gewünschten Eigenschaften des bzw. der zu gewinnenden Wirkfaktoren ausgesucht werden. Die Nukleinsäuren, die zur Herstellung des Trennmittels eingesetzt werden, werden ihrerseits aus einem Substrat der eingangs genannten Art isoliert. Hierbei ist es zweckmäßig, das Substrat vor der Abtrennung dieser Stoffe von unerwünschten oder unwirksamen Begleitstoffen zu befreien. Die unerwünschten bzw. unwirksamen Begleitstoffe können enzymatisch abgebaut werden, wonach die Abbauprodukte durch Dialyse von Bruchstücken oder durch Ultrafiltration entfernt werden. Hierbei werden in der Regel Produkte mit einem Molekulargewicht unter 600 Dalton beseitigt.

Die Herstellung des Trennmittels kann unter Verwendung von hochgereinigten Nukleinsäurefraktionen durch Bindung derselben an inertes Trägermaterial für die Affinitätschromatographie nach an sich bekannten Verfahren erfolgen. Es wurde gefunden, daß bestimmte trägergebundene Nukleinsäurefraktionen eine besondere Affinität zu tumorhemmenden Wirkstoffen besitzen.

Im einzelnen gliedert sich das erfindungsgemäße Verfahren in folgende Schritte:

a) die schonende Sterilisation der als Ausgangsstoffe dienenden fetalen oder juvenilen Organgewebe in pulverisierter Form durch Aufkondensieren flüchtiger Säuren oder Basen zur Inaktivierung etwaiger viraler Bestandteile bei gleichzeitiger Verbesserung der hydrophilen Eigenschaften durch partielle Lyse in molekulare Untereinheiten und die Addition von Resten der verwendeten Säuren und Basen,

b) die Abtrennung von Zellkern- und Membran-Bestandteilen aus dem Substrat,

c) den enzymatischen Abbau von Lipiden und Kohlenhydraten,

d) die Entfernung aller Bestandteile mit einem Molekulargewicht von weniger als 600 und mehr als 1 Mio. Dalton aus dem erhaltenen Reaktionsgemisch,

e) die Chromatographie der so erhaltenen wässerigen Substratlösung an trägergebundener DNS aus fetalen Geweben und/oder aus Tumorgeweben und/oder aus in vitro kultivierten Tumorzellen und

f) die Elution des von der trägergebundenen DNS zurückerhaltenen Anteils der Substratlösung, ggfs. durch Anlegung eines elektrischen Feldes entsprechend dem Stand der Technik.

Voraussetzung für die therapeutische Anwendung von Wirkstoffen aus biologischen Geweben ist die Sterilität bezüglich Viren. Das Verbot der Behandlung mit ionisierenden Strahlen bei Arzneimitteln und die Denaturierung durch chemische Behandlung macht das besonders schonendes Sterilisationsverfahren a) bedeutungsvoll. Dieses Verfahren ist auch für die Durchführung der weiteren Verfahrensschritte günstig, weil dabei gleichzeitig eine Verbesserung der hydrophilen Eigenschaften und die partielle Aufspaltung in molekulare Untereinheiten erreicht wird. Die acide bzw. alkalische Wirkung durch die Addition von Säuren und Laugen ist duch eine geeignete Pufferung leicht neutralisierbar, so daß während des Trennungsvorgangs ein optimaler pH-Wert gewährleistet wird.

An Zellkulturen und Tierversuchen konnte im Vergleich der nach dem Verfahrensschritt a) gewonnenen Präparate mit Präparationen, die nur der Gefriertrocknung unterworfen wurden, eine signifikant bessere Hemmwirkung auf Tumoren erzielt werden. Es ist deshalb anzunehmen, daß der Verfahrensschritt a) die Wirksamkeit des Endproduktes günstig beeinflußt.

Bei dem Verfahrensschritt a) werden Dämpfe von konzentrierten Säuren bzw. Basen, vorzugsweise von konzentrierter Schwefelsäure, Essigsäure, Ameisensäure oder Diäthylamin und/oder Mischungen von Persäuren mit den entsprechenden konzentrierten Säuren verwendet, wobei man die pulverisierten Substrate bzw. Konzentrate mit der zur Beeinflussung verwendeten Substanz im Vakuum bei Raumtemperatur ohne zusätzliche Erwärmung bedampft und den Dampf auf dem Substrat kondensiert. In der deutschen Patentschrift 1 090 821, auf die hier Bezug genommen wird, ist die Verfahrensweise im Hinblick auf einen partiellen Aufschluß des Ausgangsmaterials beschrieben. Es wurde nun gefunden, daß sich dieses Verfahren bei Verwendung von Stoffen mit sterilisierenden Eigenschaften hervorragend für Sterilisierung eignet. Perverbindungen sind besonders bevorzugt.

Es konnte nun in Versuchen mit Organpulvern aus virusinfizierten Hühnerembryonen nachgewiesen werden, daß mit einer Modifizierung des Verfahrens zum gesteuerten chemischen Aufschluß von Organen für therapeutische Zwecke (Deutsche Patentschrift 1 090 821) Sterilität erreicht wird. Das Verfahren wurde dadurch modifiziert, daß die Substrate bei einem Restwassergehalt von 5 bis 30% gegenüber einem Normalwassergehalt von 60 bis 87% von Frischgeweben, der Einwirkung von Säuredämpfen im Vakuum ausgesetzt werden, denen die entsprechenden Persäuren, besonders von Ameisensäure, Essigsäure oder Schwefelsäure, in einem Mischverhältnis von 1 Teil Persäure zu 1 bis 4 Teilen des entsprechenden Säureanhydrids zugesetzt sind. Auch können Dämpfe von Säuren verwendet werden, denen bei der Herstellung eine geringere Menge von Wasserstoffperoxyd zugesetzt wird, als zur Herstellung der Persäure erforderlich wäre, z.B. zu 10 Teilen einer 98%igen Ameisensäure, 0,1 bis 0,8% von 30%igem Wasserstoffsuperoxyd zusammen mit 0,5% konzentrierter Schwefelsäure, anstatt 10 Teilen konzentrierter Ameisensäure mit 1 Teil Wasserstoffsuperoxyd und zu 10 Teilen Essigsäureanhydrid nur 0,5 bis 4 Teile frisches 30%iges Wasserstoffperoxyd anstatt 5 Teile desselben. Der Vorteil dieses Verfahrens ist, daß die "partiellen" Persäuren weniger aggressiv und besser manipulierbar, d.h. weniger explosiv sind.

Bei der Bedampfung des Substrates wird die Menge der Säure, die auf dem Substrat kondensiert, zweckmäßigerweise so gehalten, daß sie der Einwirkung einer 0,5 bis 2%igen Persäure entspricht. Der Kondensationsvorgang kann, wenn erforderlich, mehrmals wiederholt werden, indem man das Vakuum kurz absenkt, bevor man erneut die Säuredämpfe einströmen und auf dem Substrat kondensieren läßt. Die Einwirkung kann bei nicht begrenzten Säuremengen einige Minuten, und bei Säuremengen, die auf die Substratmenge abgestimmt sind, bis zu mehreren Stunden betragen.

Der Nachweis der Sterilität des Substrates erfolgt durch die bekannten Methoden der Mikrobiologie und Virologie über die Züchtung der Viren in Hühnereiern oder in Zellkulturen. Der Nachweis der noch erhaltenen biologischen Wirkung der Präparate erfolgt durch Bioassay an Zellkulturen (vgl. V. Paffenholz und K. Theurer: Einfluß von makromolekularen Organsubstanzen auf menschliche Zellen in vitro - s.o.) sowie durch Tierversuche.

Das Verfahren führt zu keinen toxisch wirkenden Produkten, wie z.B. Heterozyklen, weil die erhaltenen Bruchstücke wegen des fehlenden Wassers nicht miteinander reaggregieren oder konjugieren. Die in geringem Ausmaße entstehenden Peroxyde bewirken in den Substraten bei der therapeutischen Anwendung eine erwünschte Reizwirkung, ähnlich wie bei der therapeutischen Anwendung von Ozon. Auch läßt sich bei diesem Verfahren die nicht zur Reaktion gekommene Säure durch Erhöhung des Vakuums absaugen. Sonst kann auch eine Neutralisierung durch Einblasen von Alkalidämpfen oder von Merkaptoäthanol, und erneutes Absaugen erfolgen. Durch die dadurch ausgelöste Reduktionswirkung läßt sich die vorausgegangene Oxydationswirkung zum Teil wieder aufheben.

Es ist auch möglich, gefrorenes, nicht getrocknetes Gewebepulver, zunächst bis zum Erreichen der gewünschten Restfeuchtigkeit durch Gefriertrocknung oder in Anwesenheit hygroskopischer Stoffe bzw. von konzentrierter Schwefelsäure vorzutrocknen, bevor man den Sterilisationsvorgang einleitet. Trockengewebe mit einer zu geringen Restfeuchtigkeit können durch Einleiten von Wasserdampf oder Anfeuchten vorbehandelt werden.

Über die Peressigsäure als Möglichkeit der "Kältesterilisation" bei der Aufbereitung thermosensibler Instrumente haben K. Bansemir, H. Bellinger, K. Disch und W. Kästner in "Hygiene und Medizin" 4 (1979) 311-316 publiziert. Dabei wurde der sterilisierende Effekt einer 1 bis 2%igen Lösung auf Papova-Viren, Entero-Viren, Poliomyelites Typ I, als auch Coxsackie-Viren Typ B III und Hepatitis-B-Viren nachgewiesen. Auch wurde über toxikologische Untersuchungen berichtet. Die Anwendung von Säurelösungen ist jedoch nicht mit dem vorliegenden Verfahren identisch, weil hier die Anwendung bei Raumtemperatur in Form von Säuredämpfen erfolgt, und beim Trocknungsvorgang die nicht zur Reaktion gekommene Säure wieder durch Absaugen beseitigt wird.

Die Verfahrensschritte b) bis d) dienen zur Vorbereitung der Abtrennung wirksamer Bestandteile aus dem aufzuarbeitenden Substrat und zur Vorbereitung des aufzutrennenden Substrates. Für die Vorbereitung der affinitätschromatographischen Apparatur (Verfahrensschritt e)) werden die DNS-Bestandteile von fetalen Zellen oder Tumorzellen benötigt. Zu deren Gewinnung wird z.B. die fraktionierte Schwerkrafttrennung von Zell- bzw. Gewebehomogenaten bzw. Suspensionen von entsprechenden Trockenpulvern bis zum 100.000 g Überstand verwendet (vgl. V. Allfrey: Isolation of subcellular components: The Cell: Vol. I; I. Brachet und A.E. Mirsky, Editors, Academic Press, London, 1959 - National Cancer Institute Monograph 21: Development of Zonal Centrifuges and Auxillary Systems for Tissue Fractionation and Analysis: National Cancer Institute Bethesda, Maryland, USA, 1966.

Zur weiteren Isolierung werden die einzelnen Fraktionen aus Zellkernen mit Enzymen behandelt z.B. mit Ptyalin und Maltase sowie mit Beta- oder Gamma-Amylase zum Abbau von Sacchariden, mit Amyloglucosidase zum Abbau von Glykogen, mit Lipasen zum Abbau von Lipiden und mit Proteinasen bzw. Peptidasen, wie z.B. Pepsin, Papain, Trypsin, zum Abbau von Eiweißkörpern (Literatur über Isolierung von DNS aus Zellen: W. Meinke, D.A. Goldstein, M.R. Hall: Anal. Biochem. 58, 82 (1974); G.G. Markov, I.G. Ivanov: Anal. Biochem., 59, 555 (1974); I.G. Ivanov, P. Venkov, G.G. Markov: Preparative Biochem., 5, 219 (1975)).

Die Abbauprodukte der enzymatischen Reinigung können durch Dialyse bzw. Ultrafiltration < 600 Mol. Gew. beseitigt werden (B.L. Williams und K. Wilson: Praktische Biochemie - Grundlagen und Techniken, S. 106: Georg Thieme Verlag, Stuttgart (1978)). Bei der Isolierung darf das Ionenmilieu und pH des

Substrats nur soweit verändert werden, als keine Denaturierung der Wirkstoffe eintritt.

Zur Vorbereitung der affinitätschromatographischen Trennung (Arbeitsschritt e)) wird die entsprechende DNS-Fraktion an inertes Trägermaterial, möglichst durch kovalente Bindung, an funktionelle, chemische Gruppen gekoppelt, ohne daß dabei reaktive, wirkungsspezifische Bindungsstellen blockiert werden. Als Trägermaterial kommen in Betracht z.B. Zellulose, Sephadex ® Hydroxylapatit, Polyacrylamid-Gel, Polyäthylengranulat, Agarose, Glasperlen, Silikonpartikel, Kieselgel, Zinkoxyd, Aluminiumhydroxyd u.a. Die Verfahren sind auch hier im einzelnen bekannt (vgl. E. Paoletti et al.: J. Biol. Chem. 249, 3273 (1974); N. Sigal et al.: Proc. Nat. Acad. Sci., USA 69, 3537 (1972); R.L. Tsai, H. Green: J. Mol. Biol., 73, 307 (1973); Sutton, D. und Kemp, J.D.: Biochemistry 15, S. 3153 (1976); Smith, I. et al.: Anal. Biochem., 48, S. 27 (1972); I.A. Lautenberger, S. Linn: J. Biol. Chem. 247, 6176 (1972). Die trägergebundene Nukleinsäurefraktion wird nun als Säulenfüllung für die Affinitätschromatographie verwendet (vgl. P. Cuatrecasas: Affinity Chromatography of Macromolecules: Advances in Enzymology, Ed. by A. Meister: 36: 29 (1972), Interscience, New York; F. Friedberg: Chromatography Reviews 14: 121 (1971). Dabei wird eine Apparatur als Kombination von Chromatographie und Elektrophorese in Art der "Free-Flow-Electrophoresis" verwendet (N. Seiler, I. Thobe, G. Werner: Elektrophorese im trägerfreien Pufferstrom: Z. Physiol. Chem. 351, 865 (1970); K. Hannig: Z. Anal. Chem. 338, 211 (1964)).

Die Gewinnung der Hemmfaktoren erfolgt aus dem sekundären Eluat. Sobald die Packung der trägergebundenen Fraktion durch Absorption des zu trennenden Substrats gesättigt ist, wird die Apparatur mit Pufferlösung geringerer Ionenstärke durchgespült. Danach erfolgt die sekundäre Elution der absorbierten Fraktion. Diese Elution kann durch Erhöhung der Ionenstärke und Veränderung des pH des verwendeten Puffers erfolgen. Besonders schonend und vorteilhaft ist eine neuartige Methode durch Anlegung eines elektrischen Gleichstromfeldes oder eines Wechselfeldes geeigneter Frequenz. Diese Art der Elution hat den Vorteil, daß die Apparatur nach erneuter Durchspülung mit der primär zur Trennung verwendeten Pufferlösung erneut verwendet werden kann. Auch die Einwirkung eines elektro-magnetischen Feldes senkrecht zur Durchflußrichtung der Pufferlösung, ggfs. in Kombination mit der Elektrophorese dient diesem Zweck. Die Auftrennung des Substrats in der nach c) wie vorstehend beschrieben vorbereiteten Trennapparatur erfolgt zweckmäßigerweise durch getaktete Proteinaufgabe und Elutionsschritte. Dabei werden in einem ersten Schritt die nichtaffinen Komponenten gewonnen und nach einer anschließenden Reinigungsspülung der Apparatur sekundär die Elution des absorbierten Wirkfaktors, wie beschrieben, durchgeführt. Das sekundäre Eluat enthält die gewünschten Wirkkomponenten.

Zur Gewinnung der tumorhemmenden Faktoren wird die Trennapparatur mit trägergebundener DNS aus fetalen Geweben und/oder aus verschiedenen Tumorgeweben konditioniert. Der absorbierende Faktor kann dabei aus einer einzelnen Ausgangssubstanz oder aus einer Kombination verschiedener Substanzen, wie z.B. aus verschiedenen Tumorarten und Metastasen, wie z.B. aus soliden Tumoren und aus leukämischen Zellen, die auch in Zellkulturen gewonnen werden, ggfs. kombiniert mit fetalen Geweben bestehen.

Die so gewonnenen angereicherten Faktoren können in wässerigen Lösungen oder inkorporiert in Liposomen bzw. in einer Wasser in Öl-Emulsion (vgl. deutsche Patentschriften 2 650 502, 2 656 333 sowie 2 640 707) in der wässerigen Phase angewandt werden. Weitere Merkmale und Vorteile von bevorzugten Ausführungsformen der Erfindung ergeben sich aus den nachfolgenden Beispielen.

**Beispiel 1**

Aufschluß und Sterilisation von Trockenpulver aus Leber durch Schwefelsäure im Vakuum.

100 g feinpulverisiertes Trockenpulver aus Leber werden verteilt auf einer Petrischale in einer Schichtdicke von 0,5 cm in einen Exsikkator gebracht, der wahlweise mit einem Kältekondensator über ein Pumpensystem für Hochvakuum, gleichzeitig aber auch mit einem kommunizierenden Vakuumgefäß in Verbindung steht, das sich durch ein Ventil von dem Hauptexsikkator absperren läßt.

Es wird nun zunächst in das kommunizierende Vakuumgefäß konzentrierte Schwefelsäure bei Zimmertemperatur eingefüllt und das Verbindungsventil zum Exsikkator abgesperrt. Um eine etwaige Restfeuchtigkeit durch hygroskopische Wirkung des Trockenpulvers zu beseitigen, wird nun zunächst mit Hilfe des Kältekondensators, der mit Aceton-Kohlensäure-Schnee unterkühlt wird, Wasser aus dem Gewebepulver sublimiert und anschließend unter Abschaltung dieses Kondensators mit Hilfe eines Hochvakuumaggregates so lange abgesaugt, bis der Druck im Exsikkator $10^{-4}$ Torr erreicht. Danach wird das Ventil zu dem kommunizierenden Gefäß geöffnet, so daß nun Schwefelsäure in dampfförmigem Zustand in den Exsikkator eindringen kann.

Das Vakuum fällt jetzt auf den Dampfdruck der Schwefelsäure ab. Nun wird die Verbindung zum Rezipienten der Säure unterbrochen, vorher aber die kommunizierenden Rezipienten von dem Pumpenaggregat getrennt. Durch Einblasen von Stickstoff in den Exsikkator wird das Vakuum über dem Leberpulver nur geringgradig weiter verschlechtert. Dadurch kondensiert der Schwefelsäuredampf auf dem Leberpulver. Nach einer gewissen Einwirkungzeit wird die Verbindung zum Pumpensystem und zum Rezipienten für die Säure wieder geöffnet und nach Erreichen des Dampfdruckes der Schwefelsäure der Vorgang zweimal wiederholt. Zum Schluß wird dann die Verbindung zwischen den beiden kommunizierenden Gefäßen nicht wiederhergestellt, sondern lediglich zwischen Exsikkator und dem Pumpensystem. Es wird nun so lange abgepumpt, bis alle Schwefelsäure aus dem Exsikkator entfernt ist, die nicht chemisch zur Reaktion gekommen ist. Auf diese Weise kann auch die Wasserstoffionenkonzentration des Trockenpulvers aus Leber auf der sauren Seite beliebig verändert werden. Je intensiver und länger die Absaugung erfolgt, um so mehr wird der pH-Wert neutral.

**Beispiel 2**

Abtrennung unwirksamer Bestandteile.

1 g der nach Beispiel 1 gewonnenen feinpulverisierten Trockensubstanz aus Leber wird in 100 ml phosphatgepufferter isotoner NaCl-Lösung (pH 7,4) durch Turbomixer bei 6-10°C homogenisiert und bei 200 g 10 Minuten zentrifugiert. Der Überstand enthält die zytoplasmatischen Zellbestandteile; das Sediment die Zellkern- und Membranfraktion. Sediment und Überstand werden getrennt weiter aufgearbeitet.

a) Der Überstand wird mit 40 ml 5 mM Tris (-Hydroxymethyl) -Aminomethan in 40 mM NaCl mit 10 ml Natriumtaurocholatlösung (80 mg/ml) versetzt und auf pH 9,2 eingestellt. Nach Zugabe von 2,5 ml Enzymsuspension (Lipase) wird zwei Stunden bei 37°C inkubiert. Dadurch werden die Lipide abgebaut.

Zum Abbau der Polysaccharide zu Glukose wird eine Konzentration von 10 mg Substrat/ml mit Na-Acetat auf einen pH-Wert von 4,8 eingestellt. Nach Zugabe von Amyloglukosidase wird bei 40°C zwei Stunden inkubiert. Anschließend wird gegen phosphatgepufferte physiologische NaCl-Lösung (pH 7,4) 24 Stunden dialysiert oder das Substrat ultrafiltriert mit einer Trenngrenze Mol. Gew. 600.

b) Zur Isolierung der DNS aus dem Sediment wird dieses in 20 ml 0,24 M $Na_3PO_4$ (pH 6,8) mit 1% SDS (Natriumdodecylsulfat), 8 M Harnstoff und $10^{-3}$ M ÄDTA (Äthylendiamintetraessigsäure) suspendiert und dieser Rohextrakt auf eine 30 X 2,5 cm-Säule mit Hydroxyapatit ($Ca_{10}(PO_4)_6(OH)_2$) gegeben. RNS (Ribonukleinsäuren), Proteine und Polysaccharide werden mit 0,24 M $Na_3PO_4$ (8 M Harnstoff) eluiert, während DNS selektiv in einem zweiten Eluat mit 0,48 M $Na_3PO_4$-Puffer gewonnen wird.

**Beispiel 3**

Kopplung von DNS an Trägermaterial.
Die Ankopplung der DNS an Zellulose als Trägersubstanz für die Affinitätschromatographie:
Eine DNS-Lösung (1-2 mg/ml in 0,01 M Tris-(Hydroxymethylaminomethan-HCl)(pH 7,4) mit 0,001 M ÄDTA wird mit trockener Zellulose im Verhältnis 1 g Zellulose zu 3 ml DNS-Lösung gemischt. Nach Trocknen über Nacht bei Raumtemperatur wird das verbleibende Wasser durch Gefriertrocknung entfernt, der dabei entstehende Puder in 20 Volumenteilen Tris-HCl suspendiert und bei 4°C ein Tag inkubiert. Die überschüssige DNS wird durch Waschen mit Pufferlösung entfernt und die DNS-Zellulose 1 : 20 mit Puffer verdünnt in eine 5 mm-Säule gefüllt.

**Beispiel 4**

Selektive Adsorption und Desorption des Wirkfaktors.
Aus einer Proteinlösung von juveniler Rinderleber, die in Analogie zu den vorhergehenden Beispielen sterilisiert und von unwirksamen Bestandteilen befreit wurde, sollen die hemmenden Faktoren angereichert werden. Man benutzt dazu eine rechteckige Flachgel-Apparatur für Affinitätschromatographie mit Elektroden an den äußeren Längsseiten. Die Apparatur wird mit der nach Beispiel 3 gewonnenen DNS-Zellulose beschickt, wobei die DNS aus fetaler Rinderleber gewonnen wurde.
Die Trennsäule wird äquilibriert mit 0,001 M ÄDTA, 0,01 M Na$_3$PO$_4$ (pH 7,4) bei 37°C. Die zu trennende Probe wird dialysiert gegen dieselbe Pufferlösung. Sie wird dann in einer Konzentration von 1 mg Protein/ml der Trennapparatur zugeführt. Nach einer Trennzeit von 8 Stunden wird die Apparatur mit Pufferlösung durchgespült. Zur sekundären Elution der von der gebundenen DNS absorbierten Faktoren wird bei weiterer Zugabe des Puffers ein elektrisches Wechselfeld von 50 KHz und 1000 V, 5 mA angelegt. Gleichzeitig wird die Ionenstärke des Puffers durch Zugabe von NaCl bis zu 1 M kontinuierlich erhöht.
Wird ein Gleichstromfeld angelegt (2000 V, 10 mA) kann gleichzeitig eine Auftrennung nach der elektrophoretischen Beweglichkeit erreicht werden durch örtlich getrennte Entnahmestellen.
Die gewonnenen Fraktionen werden an Tumorzellkulturen nach den in "Der Kassenarzt" Heft 19, Mai 1979, S. 1816-1887 angegebenen Methoden auf ihre Wirksamkeit geprüft. Damit wird die Fraktion der höchstspezifischen Aktivität ermittelt, wonach die Charge standardisiert wird.

**Beispiel 5**

Gewinnung von tumorhemmenden Faktoren.
Zur Gewinnung von tumorhemmenden Faktoren aus Leber wird die DNS-Fraktion aus einer Mischung von verschiedenen soliden Tumoren (z.B. Melanom, verschiedene Arten von Karzinom (Mamma, Prostata, Lunge, Magen, Darm, Hirn) und ggfs. Sarkomen aus mesenchymalen Geweben zusammen mit den entsprechenden Metastasen und mit in Kultur gezüchteten Leukämiezellen (lymphatischen Zellen und myeloischen Zellen)) isoliert und entsprechend Beispiel 3 an Zellulose gekoppelt und dann wie in Beispiel 4 zur Auftrennung einer Eiweißlösung aus Leber verwendet. Der Hemmfaktor wird durch sekundäre Elution gewonnen und an Tumorzellkulturen auf seine spezifische Hemmwirkung geprüft. Auf analoge Weise kann auch DNS aus Einzeltumoren, ebenso aus fetalen Geweben, insbesondere fetaler Plazenta und Leber, eingesetzt werden. Auch können Körperflüssigkeiten entsprechend aufgetrennt werden. Tumorhemmende Fraktionen lassen sich besonders auch aus dem maternen Anteil der Plazenta (Dezidua) gewinnen.

**Patentansprüche**

1. Verfahren zur Anreicherung von Wirkfaktoren zur Inhibition vor Stoffwechselvorgängen in Tumorzellen aus Zellhomogenisaten von normalen fetalen oder juvenilen Organgeweben oder von Körperflüssigkeiten, gekennzeichnet durch
a) die schonende Sterilisation von pulverisierten Ausgangsstoffen, bevorzugt durch Aufkondensieren flüchtiger Säuren oder Basen,
b) Die Abtrennung von Zellkern- und Membranbestandteilen,
c) den enzymatischen Abbau von Lipiden und Kohlenhydraten,
d) die Entfernung aller Bestandteile mit einem Molekulargewicht von weniger als 600 und mehr als 1 Mio. Dalton aus dem erhaltenen Reaktionsgemisch,
e) die Affinitätschromatographie der so erhaltenen wässrigen Substratlösung an trägergebundener DNS aus fetalen Geweben und/oder aus Tumorgeweben und/oder in vitro kultivierten Tumorzellen und
f) die Elution des von der trägergebundenen DNS zurückgehaltenen Anteils dieser Substratlösung, insbesondere durch Anlegen eines elektischen oder elektro-magnetischen Feldes.

2. Verfahren nach Anspruch 1, dadurch gekennzeichnet, daß man Verfahrensschritt a) in der Weise ausführt, daß man die Substrate im Vakuum Dämpfen vor Persäuren oder von Mischungen von Persäuren und den ertsprechenden konzentrierten Säuren aussetzt.

## Claims

1. Process for enriching active factors for the inhibition of metabolic processes in tumor cells from cell homogenates of normal fetal or juvenile organ tissues or from body fluids, characterized by

a) The gentle sterilization of pulverized initial substances, preferentially by the condensation of volatile acids or bases;

b) The separation of cellular nucleus and membrane constituents from the substrate;

c) The enzymatic breakdown of lipids and carbohydrates;

d) The removal of all constituents having a molecular weight of less than 600 daltons or more than 1 million daltons from the reaction mixture obtained;

e) The chromatography of the aqueous substrate solution thus obtained on carrier-bound DNA from fetal tissues and/or tumor tissues and/or from tumor cells cultivated in vitro;

f) The elution of the portion of the substrate solution retained by the carrier-bound DNA, in particular by applying an electrical or electromagnetic field.

2. A process in accordance with claim 1, characterized by process step a) being performed in such a manner that the substrate is exposed in a vacuum to the vapors of peracids or of mixtures of peracids and the corresponding concentrated acids.

## Revendications

1. Procédé relatif à l'enrichissement de facteurs actifs pour l'inhibition de processus métaboliques au niveau de cellules tumorales, provenant d'homogénisats cellulaires de tissus d'organe fétaux ou juvéniles normaux ou de liquides biologiques de l'organisme, caractérisé par

a) la stérilisation précautionneuse de matières de départ pulvérulentes, de préférence par application d'acides et de bases volatiles par voie de condensation,

b) la séparation des composants nucléaires et membraneux,

c) la dégradation enzymatique des lipides et hydrates de carbone,

d) l'élimination de tous les composants d'un poids moléculaire inférieur à 600 et supérieur à 1 million de Dalton du mélange réactionnel obtenu,

e) la chromatographie par affinité de la solution aqueuse de substrat ainsi obtenue sur ADN lié à une substance de support et provenant de tissus fétaux et/ou de tissus tumoraux et/ou de cellules tumorales cultivées in vitro, ainsi que l'élution de la quote-part de cette solution de substrat retenue par l'ADN lié à une substance de support, en particulier par l'application d'un champ électrique ou électro-magnétique.

2. Procédé selon revendication 1, caractérisé par le fait que l'opération a) est effectuée de telle sorte que les substrats sont exposés sous vide à des vapeurs de peracides ou de mélanges composés de peracides et des acides concentrés correspondants.

7